# EUROPEAN PATENT SPECIFICATION

(11) **EP 2 427 200 B1**
(45) Date of publication and mention of the grant of the patent: **30.04.2014**
(21) Application number: 10719037.3
(22) Date of filing: 10.05.2010
(51) Int. Cl.: A23K 1/00, A23K 1/18, A61K 35/74

(54) **FEED COMPOSITION FOR THE TREATMENT OR PREVENTION OF ENTERITIS IN FISH**
FUTTERZUSAMMENSETZUNG ZUR BEHANDLUNG ODER PRÄVENTION VON ENTERITIS BEI FISCHEN
COMPOSITION ALIMENTAIRE UTILISÉE DANS LE TRAITEMENT OU LA PRÉVENTION DE L'ENTÉRITE CHEZ LE POISSON

(30) Priority: 08.05.2009 GB 0907963; 09.11.2009 GB 0919586
(43) Date of publication of application: 14.03.2012
(73) Proprietor: Bioprotein AS, 4021 Stavanger (NO)
(72) Inventor: ROMARHEIM, Odd Helge, N-1430 s (NO); ØVERLAND, Margareth, N-1430 s (NO); MYDLAND, Liv Torunn, N-1430 s (NO); SKREDE, Anders, N-1430 s (NO); LANDSVERK, Thor, N-3677 Notodden (NO)
(74) Representative: Golding, Louise Ann
(86) International application number: PCT/GB2010/000946
(87) International publication number: WO 2010/128312

(56) References cited:
- WO-A1-03/015534
- WO-A1-2005/002606
- WO-A2-03/072133
- European Commission: "Report of the Scientific Committee on animal nutrition on the use in animal feed of protein-rich biomass derived largely from cells of methanotrophic bacteria grown using natural gas as a carbon source" 22 October 1999 (1999-10-22), XP002595438 Retrieved from the Internet: URL:http://ec.europa.eu/food/fs/sc/scan/ou t35_en.pdf [retrieved on 2010-08-05]
- AAS ET AL: "Effects of diets containing a bacterial protein meal on growth and feed utilisation in rainbow trout (Oncorhynchus mykiss)" AQUACULTURE, ELSEVIER, AMSTERDAM, NL LNKD- DOI:10.1016/J.AQUACULTURE.2006.07.033, vol. 261, no. 1, 6 November 2006 (2006-11-06), pages 357-368, XP005848603 ISSN: 0044-8486
- AAS TURID SYNNOVE ET AL: "Feed intake, growth and nutrient utilization in Atlantic halibut (Hippoglossus hippoglossus) fed diets containing a bacterial protein meal" March 2007 (2007-03), AQUACULTURE RESEARCH, VOL. 38, NR. 4, PAGE(S) 351-360 , XP002595439 ISSN: 1355-557X *Diet Composition*; page 352

## Description

This invention relates to the use of a single-cell protein material obtained from a culture of methanotrophic bacteria in the treatment and/or prevention of enteritis in fish, in particular for the prevention or amelioration of soybean-induced enteritis in carnivorous fish, e.g. salmonids such as Atlantic salmon.

Feed components account for around one third of the total cost of farming fish such as salmon and trout. Fishmeal has traditionally been the major protein source in feed for carnivorous fish. However, the price of fishmeal is steadily rising and the predicted increase in aquaculture production suggests that the world's supply of fishmeal will not be sufficient to meet future demand. Alternative sources of nutrients are therefore required to replace at least part of the fish meal fraction of aquafeeds.

The attention of the aquaculture industry has recently been drawn to plant-based ingredients, in particular plant protein sources, due to their ready availability and low price. However, such materials present a number of problems which limit their widespread use in aquaculture.

The most widely available plant protein commodity, soybean meal, is marketed as a feedstuff of high nutritional value for farmed animals, including salmon and trout, and is available as full-fat soybean meal (i.e. non-reduced oil content) and defatted soybean meal (reduced oil content) forms. In Atlantic salmon and rainbow trout, however, even at low levels in the diet soybean meal causes enteritis (a damaging inflammatory reaction of the distal intestine) which is a major ethical challenge and in turn causes impaired growth and feed utilisation (Baeverfjord and Krogdahl, Journal of Fish Diseases (1996), 19: 375-387). This condition is often referred to as "soybean meal induced enteritis" and is the main reason for low or no inclusion of soybean meal in diets for salmon and trout. The condition can also be induced by other plant materials, such as pea protein concentrate (Penn et al., XIII International Symposium on Fish Nutrition and Feeding, Florianòpolis, Brazil (2008), Book of Abstracts, p. 86).

Although the specific effects of alternative protein sources on the digestive physiology of fish have been most closely studied in the case of soybean products in feed for farmed salmonids, a recent report by the Norwegian Scientific Committee suggests there could be similar responses to other plant protein sources used by the fish feed industry. It is thus anticipated that other plant materials, such as pea protein concentrate, either alone or in combination, will show the same damaging effects as soybean meal when fed to fish as a replacement for fishmeal or other non enteritis-causing ingredients.

The severity of the morphological changes observed on feeding plant-derived products, such as soybean meal, to fish depends on the level of their inclusion in the diet. For example, enteritis of the distal intestine can be detected in salmon fed a diet containing as little as 10% by weight of soybean meal, with diets comprising 15% or more soybean meal (values based on total dietary protein) giving rise to severe morphological changes (Krogdahl et al., Aquaculture Nutrition (2003), 9: 361-371). Defatted soybean meal induces severe morphological changes in the distal intestine of Atlantic salmon and rainbow trout. Similar changes are observed on feeding soybean meal to other fish, such as gilthead bream, sea bream, common carp, Asian sea bass and channel catfish, although these are somewhat less severe.

The condition of "soybean meal induced enteritis" is not caused by the proteins in the feed, but rather by one or more alcohol-soluble components in the non-protein fraction. Protein concentrates and isolates produced from plant-based materials (e.g. soybean) following alcohol extraction can therefore be used without causing enteritis. Such products are, however, very expensive.

Thus, whilst alternative plant protein sources such as soybean meal represent promising candidates for partial replacement of fishmeal in aquaculture feeds, at present they cannot be used in any significant quantity in fish feeds due to the problem of enteritis. The present invention seeks to address this problem.

The present inventors have now surprisingly found that the addition of a bacterial protein meal or "biomass" derived from a culture comprising methanotrophic bacteria (also referred to herein simply as a "bacterial protein meal") to the diet of fish can prevent or ameliorate plant-induced enteritis. In particular, it has been discovered that the addition of low to moderate levels of bacterial protein meal to fish diets which include plant-based materials at levels which would otherwise cause enteritis renders such materials safe for feeding to fish.

Bacterial protein compositions, e.g. those comprising single-cell proteins of methanotrophic bacteria, are known for use as animal feedstuffs. For example, WO 03/015534 describes the use of a single-cell protein derived from *Methylococcus capsulatus* as a feed for fish and shellfish. However, it has not previously been proposed that a methanotrophic bacterial protein meal may be used to prevent or ameliorate plant-induced enteritis in fish, e.g. soybean-induced enteritis.

Thus, in a first aspect the invention provides a biomass derived from a culture of methanotrophic bacteria for the prevention or amelioration of plant-induced enteritis in fish.

Typically, the biomass will be directly incorporated into a conventional fish feed, for example a formulated fish feed, which will also comprise the plant-based material responsible for causing the enteritis. In a further aspect the invention thus provides a fish feed comprising a biomass derived from a culture of methanotrophic bacteria in combination with an enteritis-causing plant material. As used herein, the term "fish feed" will generally be understood to be a complete food for fish, i.e. one which comprises all the necessary components of a fish diet.

In a related aspect the invention provides a fish feed component comprising (e.g. consisting essentially of) a biomass derived from a culture of methanotrophic bacteria in combination with an enteritis-causing plant material. This feed component may be provided, for example, as an admixture of said biomass and said plant material for use as an ingredient (e.g. a protein-containing ingredient) in the preparation of a fish feed.

In a related aspect, the invention further provides a fish feed or fish feed component comprising a biomass derived from a culture of methanotrophic bacteria for the prevention or amelioration of plant-induced enteritis in fish.

In a further aspect the invention provides the use of a biomass as herein described in the manufacture of an agent (e.g. in the manufacture of a fish feed or fish feed component) for the prevention or amelioration of plant-induced enteritis in fish.

In a yet further aspect the invention provides a method of preventing or ameliorating plant-induced enteritis in fish, said method comprising administering to said fish a biomass, a fish feed or a fish feed component as herein described.

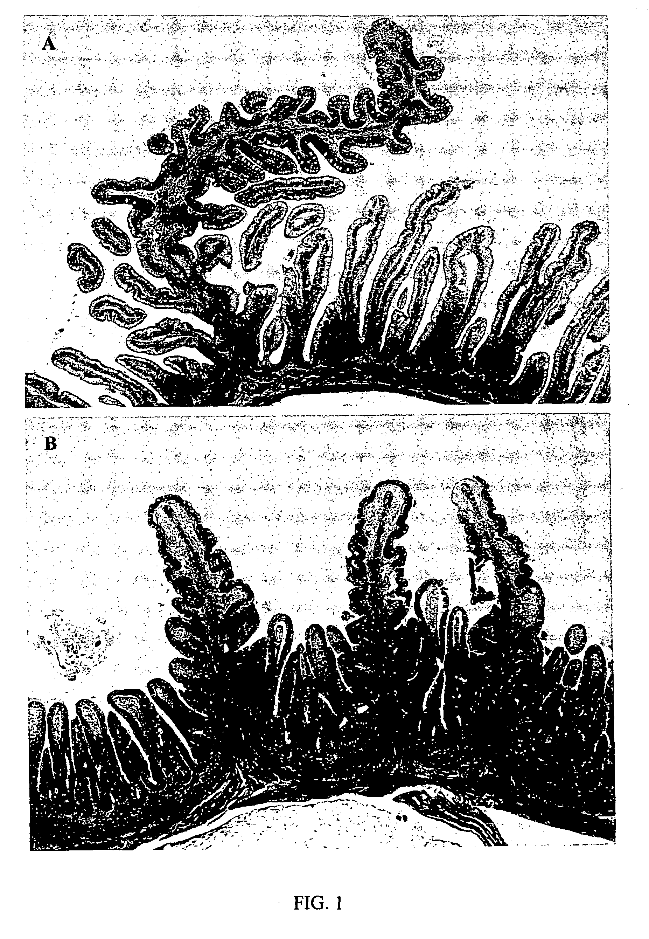

By the term "plant-induced enteritis" is meant a sub-acute inflammatory condition of the distal intestinal mucosa which arises from ingestion of a plant material. More specifically, the term is intended to refer to a condition associated with one or more of the following changes to the distal intestine: (1) widening and shortening of the intestinal folds; (2) loss of the normal supranuclear vacuolization in the absorptive cells (enterocytes) of the intestinal mucosa; (3) widening of the central lamina propria within the intestinal folds, with increased amounts of connective tissue; and (4) infiltration of a mixed leucocyte population (inflammatory cells) in the lamina propria and submucosa. These morphological changes are associated with impaired functionality of the distal intestine and are characteristic of the changes induced in fish (especially in salmon and trout) by dietary soybean meal. Such changes may readily be identified by the skilled person from a histological study of distal intestinal sections. Figure 1B is an example of the morphological characteristics of the distal intestines of Atlantic salmon when fed a diet comprising soybean meal.

Plant materials which may give rise to enteritis include whole, processed or extracted plant products, in particular plant protein materials. Such materials may, for example be derived from any of the following sources: soybean, sunflower, lupin, rapeseed, canola, cottonseed, peanut, peas (e.g. field peas), beans (e.g. faba bean), grain, barley, corn and maize.

Plant materials suitable for use in the invention are those which provide nutritional value in fish feeds and which are suitable for use as an ingredient in fish feed, e.g. suitable for the partial replacement of conventional feed ingredients such as fishmeal. Such products are those which give rise to enteritis in fish. Specific examples of materials which may result in enteritis or enteritis-like conditions in fish (depending on their level of inclusion in the diet) include soybean (full-fat, defatted, hulled or non-hulled), sunflower (e.g. defatted sunflower), lupin (e.g. dehulled lupin), rapeseed (e.g. defatted double-low rapeseed), whole field pea, pea protein concentrate, faba beans (e.g. whole and dehulled faba beans), co-products derived from biofuel production and the brewing industry, barley protein concentrate, wheat gluten and corn gluten. These ingredients may be provided without heat treatment, or may be pre-treated by methods such as roasting, toasting, microwaving, expansion, pelleting, extrusion or other known heat treatments.

Soybean products are one example of a plant-based material known to give rise to enteritis in fish. Such products are widely available in a number of different forms and all of these are considered suitable for use in the invention. These include solvent extracted soybean meal which refers to the soybean product after the extraction of part of the oil by solvents like hexane; soybean cake derived by mechanical pressure or soybean chips; soybean meal which is ground solvent extracted soybean flake or ground soybean cake, ground soybean chips, or ground soybean flakes; soybean mill feed which is the by-product resulting from the manufacture of soybean flour or grits and is composed of soybean hulls and the offal from the tail of the mill (a typical analysis by weight is 13% crude protein, 32% crude fibre and 13% moisture); soybean mill run which is the product resulting from the manufacture of dehulled soybean meal and is composed of soybean hulls and such bean meats that adhere to the hull under normal milling operations (a typical analysis by weight is 11% crude protein, 35% crude fibre and 13% moisture); soybean hulls which is the product consisting primarily of the outer covering of the soybean; and solvent extracted soybean flakes which is the product obtained after extracting part of the oil from soybeans by the use of hexane or homologous hydrocarbon solvents.

Soybean meal is commercially available in full-fat (i.e. non-reduced oil content) and defatted (reduced oil content) forms. Soybean meal is typically defatted by solvent extraction of oils using hexane or homologous hydrocarbon solvents (e.g. n-hexane or 2,3 dimethyl pentane) which are subsequently distilled off during the toasting process. Defatted soybean meal (also known as "extracted" soybean meal) typically has the following composition (% given by weight):

| | |
|---|---|
| Protein (N x 6.25) | min. 42 % |
| Fat | min. 0.2 % |
| Crude fibre | max. 8.0 % |
| Ash | max. 7.0 % |
| Water | max. 12 % or max. 13.5% |

Total carbohydrates represent the remainder, i.e. approx. 30% (of which approximately 10% is oligosaccharides, the rest is mainly non-starch polysaccharides (e.g. galactans, arabinan)).

Analysis of soybean products may be performed according to standard testing methods as adopted by the American Oil Chemists Society (AOCS), e.g. AOCS Method Ba 2a-38 (moisture), AOCS Method Ba 4e-93 (protein), AOCS Method Ba 6-84 (crude fibre) and AOCS Method Ba 3-38 (oil). Soybean meal may be dehulled (i.e. having a lower fibre content and higher crude protein content), or not dehulled.

As used herein, the term "soybean meal" refers generally to any material derived from soybean and which is suitable for use as a fish feed ingredient (with or without further processing). Suitable soybean meal for use according to the invention may be obtained from Denofa AS, Norway. Preferably the soybean meal comprises an enteritis-causing alcohol-soluble fraction, i.e. the soybean meal is capable of causing enteritis in recipient fish.

Recent studies have suggested that plant saponins may be one of the responsible factors causing symptoms of enteritis in fish. Saponins are naturally occurring amphiphilic molecules consisting of a sugar moiety linked to a steroid or triterpenoid aglycone. They are widely distributed in wild plants and are also present in many cultivated crops including soybeans and lupin seeds. Typical saponin levels in defatted soybean meal are 5-7 g/kg.

The plant material or plant source for use in the invention is thus preferably one which naturally comprises a proportion of saponins. Preferably the plant material is a plant protein-containing material, e.g. full-fat soybean meal, defatted soybean meal or pea protein concentrate (e.g. pea protein concentrate having a protein content of at least 20%). Most preferably, the material is soybean meal. In a preferred embodiment, the plant material comprises plant proteins and saponins.

In a preferred embodiment of the invention, the enteritis is induced by one of the plant materials described herein. In an especially preferred embodiment, the plant-induced enteritis is soybean-induced enteritis.

The biomass for use according to the invention is derived from or comprises a culture of methanotrophic bacteria, preferably a culture containing *Methylococcus capsulatus,* especially *Methylococcus capsulatus* (Bath). In a preferred embodiment, the biomass is derived from or comprises a major proportion of methanotrophic bacteria and a minor proportion of heterotrophic bacteria, preferably *Methylococcus capsulatus* in addition to *Ralstonia* sp., *Brevibacillus agri* and *Aneurinibacillus* sp.

Preferred bacteria for use in the biomass material include *Methylococcus capsulatus* (Bath), a thermophilic bacterium originally isolated from the hot springs in Bath, England and deposited as NCIMB 41526 (formerly NCIMB 11132) at The National Collections of Industrial and Marine Bacteria, Aberdeen, Scotland. Other bacteria suitable for use in the invention include the heterotrophic bacterium DB3, strain NCIMB 41527 (*Ralstonia* sp. formerly known as *Alcaligenes acidovorans* DB3; formerly NCIMB 13287), DB5, strain NCIMB 41525 (*Brevibacillus agri* formerly known as *Bacillus firmus* DB5; formerly NCIMB 13289) and DB4, strain NCIMB 41528 (*Aneurinibacillus* sp. formerly known as *Bacillus brevis* DB4; formerly NCIMB 13288) which each have optimum growth at a temperature of about 45°C. Details relating to the characteristics of these bacterial strains are given in WO 03/072133, the content of which is incorporated herein in its entirety. An especially preferred biomass for use according to the invention is that prepared from a microbial culture of the above strains and is available from Norferm AS, Norway under the trade name BioProtein^{®}.

The biomass for use in the invention is derived from a microbial culture which comprises a methanotrophic bacterial strain, optionally in combination with one or more species of heterotrophic bacteria, especially preferably a combination of methanotrophic and heterotrophic bacterial strains. As used herein, the term "methanotrophic" encompasses any bacterium which utilizes methane, methanol or formaldehyde for growth. The term "heterotrophic" is used for bacteria that utilize organic substrates other than methane, methanol or formaldehyde for growth.

The bacterial biomass for use in the methods herein described may be formed by growth of the bacteria on a suitable medium or substrate. The exact nature of the growth medium used to produce the biomass is not critical and a variety of suitable substrates may be used. Conveniently, the biomass may be produced by a fermentation process in which oxygen and a suitable substrate such as a liquid or gaseous hydrocarbon, an alcohol or carbohydrate, e.g. methane, methanol or natural gas, together with a nitrogen source (preferably ammonia) and a nutrient mineral solution are fed to a tubular reactor containing the microorganisms. A number of such processes are well known and described in the art, for example in WO 01/60974, DK-B-170824, EP-A-418187 and EP-A-306466, the contents of which are herein incorporated by reference.

The biomass material for use in the invention will preferably be derived from fermentation on hydrocarbon fractions or on natural gas. Especially preferred are biomass materials derived from the fermentation of natural gas. Generally in such processes, as the concentration of microorganisms increases within the fermenter, a portion of the reactor contents or broth is withdrawn and the microorganisms may be separated by techniques well known in the art, e.g. centrifugation and/or ultrafiltration. Conveniently, in such a fermentation process, the broth will be continuously withdrawn from the fermenter and will have a cell concentration between 1 and 5% by weight, e.g. about 3% by weight.

Especially preferred for use in the invention is a microbial culture comprising a combination of the methanotrophic bacterium *Methylococcus capsulatus* (Bath) (strain NCIMB 41526), and the heterotrophic bacteria DB3 (strain NCIMB 41527) and DB5 (strain NCIMB 41525), optionally in combination with DB4 (strain NCIMB 41528). The role of DB3 is to utilize acetate and propionate produced by *M capsulatus* (Bath) from ethane and propane in the natural gas. DB3 may account for up to 10%, e.g. about 6 to 8%, of the total cell count of the resulting biomass. The role of DB4 and DB5 is to utilize lysis products and metabolites in the medium. Typically, DB4 and DB5 will each account for less than 1% of the cell count during continuous fermentation.

Suitable fermenters for use in preparing the biomass are those of the loop-type, such as those described in DK 1404/92, EP-A-418187 and EP A 306466 of Dansk Bioprotein, or air-lift reactors. Other fermenters may be used in preparing the biomass and these include tubular and stirred tank fermenters.

Typically, the biomass produced from fermentation of natural gas may comprise from 60 to 80% by dry weight crude protein; from 5 to 20% by weight crude fat; from 3 to 10% by weight ash; from 3 to 15% by weight nucleic acids (RNA and DNA); from 10 to 30 g/kg phosphorus; up to 350 mg/kg iron; and up to 120 mg/kg copper. Particularly preferably, the biomass will comprise from 68 to 73%, e.g. about 70% by weight crude protein; from 9 to 11%, e.g. about 10% by weight crude fat; from 5 to 10%, e.g. about 7% by weight ash; from 8 to 12%, e.g. about 10% by weight nucleic acids (RNA and DNA); from 10 to 25 g/kg phosphorus; up to 310 mg/kg iron; and up to 110 mg/kg copper. The amino acid profile of the protein content can be expected to be nutritionally favourable with a high proportion of the more important amino acids methionine, cysteine, threonine, lysine, tryptophan and arginine. Typically these may be present in amounts of about 3.1%, 0.7%, 5.2%, 7.2%, 2.5% and 6.9%, respectively (expressed as a per cent of the total amount of amino acids). Generally the fatty acids will comprise mainly the saturated palmitic acid (approx. 50%) and the monounsaturated palmitoleic acid (approx. 36%). The mineral content of the product will typically comprise high amounts of phosphorus (about 1.5% by weight), potassium (about 0.8% by weight) and magnesium (about 0.2% by weight).

Typically, the resulting biomass will be produced in the form of a flowable aqueous paste or slurry. Generally this will consist essentially of whole cell material, although a proportion of ruptured cell material may also be present.

The product from the fermenter may be used directly (i.e. without further processing) or subjected to further processing steps before use as the biomass component of the invention. Further processing steps include homogenisation, in addition to centrifugation and/or filtration (e.g. ultrafiltration) processes whereby to reduce the water content prior to use. Suitable homogenisation methods are described, for example, in WO 01/60974, the content of which is incorporated herein by reference.

Following production of the biomass, this is generally concentrated from the fermentation medium, for example by conventional centrifugation and/or filtration methods, e.g. microfiltration or ultrafiltration. Filtration is preferably carried out at a temperature in the range of from 50 to 70°C. The size exclusion used during ultrafiltration will generally be in the range of about 100kD. However, filters having a MW cut-off in the range of from 10 to 100kD, e.g. about 20kD, may be used. Microfiltration will generally be carried out using filters in the range of 0.2µm to 0.4µm.

Concentration of the biomass may be effected by centrifugation alone. During centrifugation, the dry matter content of the biomass is typically increased from about 2 to about 15% by weight, particularly to about 5 to 18% by weight, preferably 8 to 15%, e. g. about 14% by weight. If necessary, or indeed desirable, filtration (e.g. ultrafiltration) methods may be used to further increase the solids content of the biomass. Ultrafiltration, which may advantageously be effected at a temperature of between 40 and 50°C, e. g. between 42 and 46°C, further concentrates the biomass to a product containing from 10 to 30%, preferably from 15 to 25%, e. g. from 18 to 22% by weight single-cell material. The resulting biomass will be in the form of an aqueous slurry and will typically have a solids content in the range of from 10 to 30%, preferably 15 to 25%, e. g. about 20% by weight.

The biomass for use in the invention may also be a hydrolysate or an autolysate of the microbial culture. WO 03/068002 and WO 03/068003 describe processes for preparing bacterial hydrolysates and autolysates respectively and their contents are incorporated herein in their entirety. Hydrolysis and/or autolysis steps may be carried out before or after any centrifugation and/or filtration steps. The exact order of processing steps will depend on the desired nature of the product and may be determined by one of skill in the art.

Bacterial hydrolysates may be produced by the action of one or more enzymes capable of hydrolysing (e.g. hydrolytically degrading) the cell structure and/or intracellular components of the bacterial culture, preferably an enzyme or enzyme system capable of hydrolysing the nucleic acid content of the cells. Bacterial autolysates may be prepared by incubation of the bacterial culture under carefully controlled conditions to allow the endogenous enzymes contained within the bacterial cells, such as nucleases and proteases, to digest the components of the cell. This "self-digestion" process results in the production of various degradation products of the cell which may include peptides, amino acids, nucleotides, phospholipids, fatty acids, etc. Suitable reaction conditions for hydrolysis and autolysis of the bacterial culture may be determined by one skilled in the art.

The biomass for use in the invention may also be a permeate, i.e. the soluble fraction obtained following filtration, e.g. microfiltration or ultrafiltration. A preferred permeate is obtained by homogenisation, autolysis and then ultrafiltration of the bacterial biomass. Another preferred permeate is obtained by homogenisation and then microfiltration of the bacterial biomass. To improve the yield of product, the hydrolysate or autolysate may be washed repeatedly (e. g. up to 5 times, e. g. up to 3 times) with water followed by ultrafiltration steps. Following separation of the permeate from the solid fraction retained by the filter (herein called the retentate) the solids content of the permeate may be expected to be about 1 to 8% by weight, e.g. in the range of from 1 to 3.5% by weight, especially around 2% by weight, or in the range of from 3 to 8% by weight, especially around 4.5% by weight.

Following centrifugation and/or ultrafiltration the biomass material will be a relatively viscous protein slurry or paste. Although this may be used directly in the products and methods herein described, this will usually be further processed whereby to remove excess water from the product. The choice of any additional drying step or steps will depend on the water content of the material and the desired moisture content of the final product and could be determined by the skilled person. Typically, the product will be further processed in accordance with spray drying techniques well known in the art.

The bacterial biomass or processed biomass herein described may be treated to reduce the nucleic acid content of the biomass. Methods for the reduction in nucleic acid content of bacterial biomass are known in the art and include heat shock treatments, e.g. according to the method described in Larsen and Joergensen (Applied Microbiology and Biotechnology (1996) 45, pp.137-140). A bacterial biomass, or fraction thereof, treated to reduce the nucleic acid content is referred to herein as a "nucleic acid-reduced" biomass or biomass fraction, respectively. A nucleic acid-reduced bacterial biomass preferably comprises nucleic acids (measured as the content of DNA and RNA in the biomass material relative to the other, non-solvent components) at a level of less than 40%, preferably less than 30% and especially preferably less than 20%, e.g. at a level of from 5 to 25%, of the content of nucleic acids in the bacterial biomass before nucleic acid reduction treatment. For example, a nucleic acid-reduced biomass according to the invention preferably comprises a nucleic acid content of from 1 to 4% by weight, especially from 2 to 3% by weight, especially around 2.2% by weight.

Thus, suitable derivatives of the biomass material for use according to the invention include biomass derived from a culture comprising methanotrophic bacteria by one or more of the following processes: centrifugation, filtration (e.g. ultrafiltration), homogenisation, hydrolysis and/or autolysis. Other suitable derivatives include biomass concentrates (e.g. an enriched biomass fraction following centrifugation and/or ultrafiltration), biomass permeates (i.e. the soluble fraction obtained after filtration of the biomass or biomass fractions) and nucleic acid-reduced biomass fractions. Further suitable derivatives include biomass and biomass derivatives grown on methanol, especially nucleic acid-reduced biomass derivatives grown on methanol.

In a preferred embodiment, the invention therefore provides a biomass, fish feed or fish feed component as herein defined, wherein the biomass is as described herein, e.g. derived from a culture comprising methanotrophic bacteria by one or more of the following processes: centrifugation, filtration (e.g. ultrafiltration), homogenisation, hydrolysis and/or autolysis, or wherein the biomass is a biomass concentrate, a biomass permeate or nucleic acid-reduced biomass fraction.

By the term "biomass derived from a culture of methanotrophic bacteria" is meant a nutrition-providing culture of methanotrophic bacteria as defined above, or a nutrition-providing extract or processed fraction of such a culture. By "nutrition-providing" is meant that the biomass is suitable for use as an ingredient in fish feed, e.g. suitable for the partial replacement of conventional feed ingredients such as fishmeal. The biomass or fish feed of the invention is suitable for the prevention or amelioration of plant-induced enteritis in fish that suffer from said condition.

Typically, the biomass herein described will be fed to fish in combination with the enteritis-inducing plant material, e.g. in a formulated fish feed, and is thus preferably incorporated into a conventional feed containing a plant protein source, e.g. soybean meal. Methods for mixing feed components and providing fish feeds (e.g. in extruded or pellet form) are well known in the art. Preferred forms for fish feeds of the invention include dry pelleted, expanded and extruded forms and also include moist and semi-moist forms. Alternatively, the biomass may be fed to fish separately from the enteritis-inducing plant material as a supplement to the diet.

A process for preparing a fish feed or feed component according to the invention, e.g. as described above, will generally include the admixture of the bacterial protein meal with an enteritis-causing plant material (e.g. soybean meal) and, optionally, with one or more conventional fish feed ingredients. This mixture may then be further processed. Suitable conventional ingredients and methods for preparing fish feeds and feed components are well known in the art (see also Table 2 and Example 4).

Thus, viewed from a further aspect, the invention provides a process for preparing a fish feed or feed component according to the invention, which process comprises admixing a biomass derived from a culture of methanotrophic bacteria and an enteritis-causing plant material (e.g. soybean meal). In a preferred embodiment, the process comprises admixing said biomass with one or more conventional feed ingredients.

Fish which may suffer from plant-induced enteritis include both carnivorous and omnivorous fish (although the extent to which they suffer from the condition will depend on several factors, including the fish species, inclusion level and duration of feeding the plant material, etc.). The condition is typically observed within 1 day to 4 weeks of feeding the fish on plant-derived feedstuffs. Typical levels of dietary inclusion of plant-based materials which give rise to enteritis in fish are at least 5% by weight, especially at least 8% by weight and particularly at least 10% by weight (based on the total weight of the diet). Such values may also be given based on the total weight of dietary protein and may be at least 5% by weight, preferably at least 8% by weight, e.g. in the range of 8 to 40%, preferably 10 to 25%. Plant-derived feedstuffs which can induce enteritis are as herein defined, but include soybean meal (full-fat and defatted) as well as pea protein concentrates and other enteritis-causing materials, e.g. lupin seeds.

The maximum tolerable level of plant protein in the feed according to the invention will be dependent on the species of fish and on the level of other components in the feed, especially on the level of biomass in the feed, but may readily be determined by those skilled in the art.

A conventional feed (e.g. for salmonids) may, for example, comprise (by weight): 10-60%, e.g. about 30%, fishmeal; 5-35%, e.g. about 20%, fish oil; 0-35%, e.g. about 20%, non enteritis-causing plant protein ingredients; 5-20%, e.g. about 10%, plant oils; 5-15%, e.g. about 10%, starch ingredients (e.g. wheat); and about 1% of other components (e.g. minerals, vitamins, colouring agents etc.). Examples of such feeds are given in Example 2.

In one embodiment, the fish feed according to the invention may comprise (by total weight of feed): 0-25%, e.g. 5-15%, preferably about 12% fishmeal; 1-50%, preferably 5-40%, especially 10-25%, e.g. about 15%, bacterial biomass as herein defined; and 5-50%, e.g. 10-40% or 15-30%, preferably about 20%, enteritis-causing plant material (e.g. soybean meal). In a further embodiment, the fish feed according to the invention may comprise (by total weight of feed): 0-25%, e.g. 5-15%, preferably about 10% fishmeal; 2.5-30%, preferably 5-20%, especially 10-15%, bacterial biomass as herein defined; and 5-20%, preferably 10-15%, enteritis-causing plant material (e.g. soybean meal). The fish feed of the invention will typically comprise other components for the health and nutrition of fish, such as are listed above. The quantities of these additional components may be determined by the skilled person.

Where the fish feed is intended for use in feeding salmon this may contain at least 5% by weight soybean meal, e.g. at least 10%, at least 15%, at least 20% or at least 25% by weight (based on the total weight of the diet). Where the fish feed is intended for use in feeding trout, the feed may contain at least 10% by weight soybean meal, e.g. at least 20%, at least 30% or at least 35% by weight (based on the total weight of the diet).

As will be appreciated, where other protein ingredients are present in the final feed and the bacterial biomass is not intended to provide any significant contribution to the protein content of the fish diet, it is preferable that this should be used in relatively low amounts provided that this is capable of achieving the necessary enteritis-reducing effect. On the other hand, where the biomass is intended to contribute to the protein content of the diet, this may be used in higher amounts. The actual amount of biomass which may be used will naturally be dependent on several factors, including the nature of the enteritis-causing plant material (i.e. the extent to which this is responsible for enteritis) and the amount in which this is present in the feed, the nature of the fish species, etc. Taking into account these factors, suitable amounts of the biomass may readily be determined by those skilled in the art.

In one embodiment, a fish feed of the present invention may comprise at least 0.5% by weight of bacterial biomass (as herein defined), preferably at least 0.75% by weight, e.g. at least 1, 1.2, 1.5, 2, 2.5, 3, 4 or 5% by weight. A fish feed comprising between 1.5 and 2.5% by weight of bacterial biomass is particularly preferred. Alternatively, a fish feed comprising between 1 and 50% by weight, especially between 2 and 40% by weight, e.g. between 2.5 and 30%, between 5 and 20% or between 10 and 15% by weight, is also preferred. A fish feed comprising between 20 and 50% by weight, especially between 30 and 40% by weight, bacterial biomass is also preferred. In a further embodiment, the fish feed of the present invention may comprise up to 40% by weight of enteritis-causing plant material as herein defined (e.g. soybean meal), preferably up to 30% by weight. A fish feed comprising between 5 and 40% by weight plant material, especially between 10 and 35%, e.g. from 10 to 15%, by weight or 20 and 30% by weight plant material is preferred. A fish feed comprising between 2.5 and 15% by weight of bacterial biomass and between 10 and 15% by weight of enteritis-causing plant material as herein defined (e.g. soybean meal) is especially preferred.

The ratio of plant material (e.g. soybean meal) to bacterial biomass in the feeds herein described is preferably between 1:10 and 10:1, preferably between 1:5 and 5:1, between 1:3 to 3:1 or 1:2 to 2:1, especially preferably about 1:1. In an alternative embodiment, the ratio of plant material to biomass in the feeds herein described may be less than 80:1, preferably less than 40:1, e.g. less than 16:1. For example, suitable ratios may be 8:1 to 1:4, preferably 4:1 to 1:3, especially 3:2 to 2:5. In an alternative embodiment, the ratio of plant material to biomass in the feeds herein described may be 25:1 to 2:1, especially around 4:1. A preferred ratio of plant material (e.g. soybean meal) to biomass in the feeds herein described is less than 8:1, especially less than 4:1, e.g. between 4:1 and 1:1, especially about 2:1.

In the embodiment of the invention where the bacterial biomass is provided as part of a feed component, the relative amounts of biomass and enteritis-causing plant material may be the same as those set out above in respect of the above feeds, i.e. where no further biomass or plant material are provided in the final feed (and thus the ratio of said components in the final feed will be the same as that in the feed component). Alternatively, the relative amounts of biomass and plant material in the feed component may be such that incorporation of said component into the final feed results in the ratios described above. In a particularly preferred embodiment, the ratio of plant material (e.g. soybean meal) to biomass in the feed component is 20:1 to 1:4, preferably 8:1 to 1:3. Preferred feed component may comprise a ratio of enteritis-causing plant material to biomass of around 10:1 or of around 4:1, a ratio of 10:1 1 is particularly preferred. In an alternative embodiment, a preferred feed component comprises a ratio of enteritis-causing plant material to biomass of between 4:1 and 1:1, e.g. around 4:1, around 2:1 or around 3:2.

Particularly preferred in accordance with the invention is a feed component which comprises about 10% by weight bacterial biomass and about 90% by weight enteritis-causing plant material. Also preferred in accordance with the invention is a feed component which comprises about 20% by weight bacterial biomass and about 80% by weight enteritis-causing plant material (e.g. soybean meal), or which comprises about 35% by weight bacterial biomass and about 65% by weight enteritis-causing plant material.

It is envisaged that the plant-based material herein described will be used as a replacement for fishmeal or other non enteritis-causing protein sources in fish feeds. Accordingly, the invention further provides a fish feed having a reduced fishmeal content, characterised in that some or all of the fishmeal is replaced by an enteritis-causing plant material and a bacterial biomass as herein defined. Preferably the plant material is as hereinbefore defined and is especially preferably soybean meal. Preferably at least 50% of the fishmeal may be replaced, e.g. at least 75%, at least 85% or at least 95%.

Whilst the methods herein described are applicable to any fish susceptible to plant-induced enteritis, the present invention is directed particularly at carnivorous fish, especially salmonids (of the family *Salmonidae*) and particularly salmon, e.g. Atlantic salmon (*Salmo salar*)*,* chinook salmon (*Oncorhynchus tshawytscha*) or Coho salmon *(Oncorhynchus kisutch*); trout, e.g. rainbow trout (*Oncorhynchus mykiss*); char, e.g. Arctic char (*Salvelinus alpinus*); whitefish, e.g. common whitefish *(Coregonus lavaretus*); grayling, e.g. *Thymallus thymallus;* and amberjack, e.g. *Seriola dumerili.* The invention is also applicable to bream, e.g. gilthead bream (*Sparus aurata* Linn.) and sea bream; carp, e.g. common carp (*Cyprinus carpio);* cod, e.g. Atlantic cod *(Gadus morhua*); halibut, e.g. Atlantic halibut (*Hippoglossus hippoglossus);* turbot, e.g. European turbot (*Psetta maxima*); sea bass (e.g. Asian sea bass); tilapia, e.g. of the genus *Oreochromis;* and catfish, e.g. channel catfish (*Ictalurus punctatus*) and members of the family *Pangasiidae* (e.g. *Pangasius bocourti).*

The invention will now be described in more detail in the following nonlimiting Examples and with reference to the accompanying Figures in which:
Figure 1 shows cross-sections of the distal intestinal walls of Atlantic salmon fed in accordance with the procedure in Example 4. Figure 1A is a sample from a fish fed with a diet comprising 13% by weight fishmeal, 20% by weight soybean meal and 30% by weight bacterial protein meal (and which shows no signs of enteritis). Figure 1B is a sample from a fish fed with a fishmeal diet comprising 41 % by weight fishmeal and 20% by weight of soybean meal (and no bacterial protein meal) which displays a morphology highly characteristic of soybean-induced enteritis.
Figure 2 shows a grading of leukocytes accumulation in the lamina propria of the distal intestinal walls of Atlantic salmon fed in accordance with the procedure in Example 5.
Figure 3 shows a grading of epithelia of the distal intestinal walls of Atlantic salmon fed in accordance with the procedure in Example 5.
Figure 4 shows a grading of the intestinal folds (reduced atrophy) of the distal intestinal walls of Atlantic salmon fed in accordance with the procedure in Example 5.
Figure 5 shows a grading of levels of oedema in the distal intestines of Atlantic salmon fed in accordance with the procedure in Example 5.

### Example 1 - Fish feed components

Table I shows typical compositions of various ingredients which may be formulated to produce fish feeds:

**Table 1**

| | Fishmeal | Wheat | Soybean meal | Bacterial protein meal |
|---|---|---|---|---|
| Ingredients, g kg⁻¹ | | | | |
| Crude protein | 677.50 | 134.63 | 454.56 | 656.25 |
| Dry matter | 913.24 | 861.95 | 881.36 | 921.27 |
| Ash | 127.48 | 13.18 | 58.02 | 65.00 |
| Crude lipids | 80.48 | 13.43 | 9.30 | 109.65 |
| Starch | 5.80 | 598.00 | 4.00 | 20.00 |

### Example 2 - Complete fish feeds

Table 2 shows the formulation of various fish diets. Diet 1 is a fishmeal (FM) diet; diet 2 is a 20% defatted soybean meal (SBM) diet; and diet 3 is a combination of 13% fishmeal, 20% defatted soybean meal and 30% bacterial protein meal (BPM) diet (% are by weight based on the total diet). Diet 3 is in accordance with the invention.

**Table 2**

| | FM diet | SBM diet | BPM-SBM diet |
|---|---|---|---|
| Main ingredients, g kg⁻¹ | | | |
| Fishmeal¹ | 591.4 | 412.4 | 127.4 |
| Bacterial protein meal² | - | - | 300.0 |
| Defatted soybean meal³ | - | 200.0 | 200.0 |
| Fish oil⁴ | 233.0 | 224.0 | 216.0 |
| Wheat | 170.0 | 158.0 | 151.0 |
| Vitamin, mineral, marker and astaxanthin mixture⁵ | 5.6 | 5.6 | 5.6 |

| | | | |
|---|---|---|---|
| ¹ Norse LT-94^{®}, low-temperature dried fishmeal (Norsildmel, Bergen, Norway). ² BioProtein^{®} (Norferm AS, Stavanger, Norway). ³ Extracted and toasted soybean meal (Denofa, Fredrikstad, Norway). ⁴ Silfas, Karmsund, Norway. ⁵ Per kg diet: vitamin A: 2500 IU; vitamin D₃: 1500 IU; vitamin E: 200 mg; vitamin K₃: 10 mg; vitamin B₁: 15 mg; vitamin B₂: 25 mg; vitamin B₃: 75 mg; vitamin B₅: 30 mg; vitamin B₆: 15 mg; vitamin B₉: 5 mg; vitamin B₁₂: 0.02 mg; vitamin C: 125 mg; biotin: 0.25 mg; Ca: 1.1g; Zn: 120 mg; Mn: 15 mg; Cu: 5 mg; Co: 1 mg; I: 3 mg; astaxanthin: 175 mg (F. Hoffmann-La Roche, Basel, Switzerland); Yttrium oxide 99,9 % purity: 100 mg (Metall Rare Earth Limited, Shenzhen, Guangdong, China). | | | |

### Example 3 - Complete fish feeds

Table 3 shows the formulation of various fish diets. The FM diet is a high quality fishmeal diet. Diets 1-6 are in accordance with the invention and comprise varying levels of bacterial protein meal and a fixed amount of defatted soybean meal which are used to replace, in part, the fishmeal component of the FM diet.

**Table 3**

| | FM diet | Diet 1 | Diet 2 | Diet 3 | Diet 4 | Diet 5 | Diet 6 |
|---|---|---|---|---|---|---|---|
| Main ingredients, g kg⁻¹ | | | | | | | |
| Fishmeal¹ | 642.4 | 459.4 | 436.4 | 412.4 | 364.4 | 316.4 | 269.4 |
| Bacterial protein meal² | 0 | 0 | 25 | 50 | 100 | 150 | 200 |
| Defatted soybean meal³ | 0 | 200 | 200 | 200 | 200 | 200 | 200 |
| Fish oil⁴ | 189 | 184 | 183 | 182 | 181 | 180 | 178 |
| Wheat | 163 | 151 | 150 | 150 | 149 | 148 | 147 |
| Vitamin, mineral, marker and astaxanthin mixture⁵ | 5.6 | 5.6 | 5.6 | 5.6 | 5.6 | 5.6 | 5.6 |

| | | | | | | | |
|---|---|---|---|---|---|---|---|
| ¹⁻⁵ as in Example 3. | | | | | | | |

### Example 4 - Feeding study

### Diets

Three diets were formulated in accordance with Table 2 of Example 2. These comprised: a control diet based on high-quality fishmeal (FM diet), a diet with 200g kg⁻¹ defatted soybean meal (SBM diet) and a diet containing a combination of 300g kg⁻¹ bacterial protein meal (BPM) and 200g kg⁻¹ SBM (BPM-SBM diet). BPM and SBM were used to replace FM and the amount of wheat and fish oil in each diet was altered to provide diets having a roughly equal ratio between crude protein, crude lipids and starch. The addition of vitamins and minerals was constant for all diets, and yttrium oxide was used as indigestible marker for determination of apparent digestibility of nutrients. The diets were produced by extrusion technology at the Center for Feed Technology, Ås, Norway. All dry ingredients were ground in a Münch hammer mill (HM 21.115, Wuppertal, Germany) with a 1 mm screen. The meal mixtures were conditioned (97 ± 1.9 °C; 26 ± 1.4 % moisture (mean ± S.E.M.)) in a double shaft conditioner (BCTC 10, Bühler, Uzwil, Switzerland) before extrusion (110 ± 5.9 °C at die) by a five section Bühler twin-screw extruder (BCTG 62/20 D, Bühler, Uzwil, Switzerland) with a 3 mm die.

### Fish and rearing conditions

The fish trial was carried out at AKVAFORSK, Sunndalsøra, Norway, with Atlantic salmon *(Salmo salar*) with 133.5 g mean initial weight. Each diet was fed to three groups of fish kept in 1 m³ fibreglass tanks supplied with seawater (8.6 °C; 32.5 g l⁻¹ salinity). The feeding trial lasted for 80 days. All tanks had light 24 hour a day and the diets were fed every 10 mins, 24 hours a day, using electrically driven disc feeders. Uneaten feed was sieved from the outlet water of each tank and feed intake was monitored.

Since the commercial diet used to rear the fish might have contained soybean meal, all fish were fed the FM diet for at least four weeks prior to the trial to avoid possible interactions with the experimental diets containing soybean meal. Four weeks pre-feeding is considered to be sufficient for a complete rebuilding of the intestinal folds, if damaged.

### Weighing and sampling for analysis

Random samples of the individual protein rich feed ingredients and feeds were ground and frozen prior to chemical analysis. The fish were bulk-weighed at the start of the experiment, and individually weighed at the end of the experiment (day 80). At termination, five fish from each tank were picked at random for sampling of organs for histochemical evaluation.

The intestinal tracts of five fish from each tank were dissected for histological examination of wall tissue from pyloric caeca and from mid and distal intestine. Samples of about 5 x 5 mm were obtained from the mid part of the intestinal sections and fixed in 4% phosphate buffered formaldehyde (10% formalin). Formalin fixed tissue was routinely dehydrated in ethanol 48 hours pre-sampling, equilibrated in xylene and embedded in paraffin according to standard histological techniques. Sections of approximately 5 µm were cut and stained with haematoxylin and eosin before examination under a light microscope. Intestinal morphology was evaluated according to the following criteria described for soybean meal-induced enteritis in Atlantic salmon (Baeverfjord and Krogdahl, supra): (1) widening and shortening of the intestinal folds; (2) loss of the supranuclear vacuolization in the absorptive cells (enterocytes) in the intestinal epithelium; (3) widening of the central lamina propria within the intestinal folds, with increased amounts of connective tissue; and (4) infiltration of a mixed leucocyte population in the lamina propria and submucosa.

### Chemical and biochemical analyses

Dry matter, crude protein (Kjeldahl N x 6.25) and ash were analysed according to standard procedures of AOAC International, US (AOAC, 1990), crude lipid was determined after hydrolysis with petroleum ether on an Accelerated Solvent Extractor (ASE200) from Dionex (Sunnyvale, CA, USA), and starch (Total Starch Assay Kit [AA/AMG], Megazyme International Ireland Ltd., Wicklow, Ireland) determined as total glucose after hydrolysis with glucosidase.

### Calculations and statistical analyses

Feed intake of a period was calculated as DM_{feed} x BW₀⁻¹, where DM_{feed} is the consumption of feed dry matter and BW₀ represents the initial body weight (tank means) of the period. Thermal growth coefficients were calculated as 1000 x (BW₁^{1/3} - BW₀^{1/3})/ d°, where BW₁ represent the final body weight and d° represents thermal sum (mean daily temperature x days of the period). Feed conversion ratio (FCR) was calculated as DM_{feed} x (BW₁ - BW₀)⁻¹.

Statistical analyses were carried out using JMP from SAS Institute Inc. Significant (P<0.05) differences among means were ranked. The results are presented as mean ± standard error of mean (s.e.m.).

### Results

Table 4 shows the analysed chemical composition of the various diets.

**Table 4**

| | FM diet | SBM diet | BPM-SBM diet |
|---|---|---|---|
| Dry matter (DM), g kg⁻¹ | 951 | 954 | 949 |
| Chemical composition, g kg⁻¹ | | | |
| DM | | | |
| Crude protein | 455 | 438 | 446 |
| Crude fat | 284 | 266 | 258 |
| Starch | 110 | 113 | 122 |
| Ash | 83 | 78 | 56 |
| Gross energy, MJ kg⁻¹ | 24.2 | 24.5 | 24.6 |

Table 5 shows the least-square means for feed intake, growth and feed conversion (n=3).

**Table 5**

| Diet | FM diet | SBM diet | BPM-SBM diet | Pooled s.e.m. | P- value |
|---|---|---|---|---|---|
| Feed intake, g DM (kg initial weight)⁻¹ | 1239 | 1080 | 1075 | 43.1 | 0.060 |
| Mean weight, g fish⁻¹ | | | | | |
| Start | 133^{b} | 132^{a} | 135^{c} | 0.2 | <0.001 |
| End | 362^{b} | 319^{a} | 328^{a} | 8.0 | 0.020 |
| Thermal growth coefficient (TGC) | 2.90^{b} | 2.49^{a} | 2.53^{a} | 0.076 | 0.017 |
| Feed conversion ratio, g DM intake (g body weight gain)⁻¹ | 0.72^{a} | 0.76^{b} | 0.75^{b} | 0.007 | 0.011 |

| | | | | | |
|---|---|---|---|---|---|
| ^{a,b,c} Different superscript letters within a row indicate significant (P<0.05) differences among dietary treatments. | | | | | |

Table 6 shows the results of a visual classification from histology of distal intestinal sections.

**Table 6**

| Diet | FM diet | SBM diet | BPM-SBM diet |
|---|---|---|---|
| Classification of distal intestine (No. of fish out of 15) | | | |
| Normal intestine | 15 | 0 | 15 |
| Minor inflammation | 0 | 0 | 0 |
| Moderate to severe inflammation | 0 | 15 | 0 |

Figures 1A and 1B show cross-sections of intestinal walls from fish fed the BPM-SBM diet (Fig. 1A) and fish fed the SBM diet (Fig. 1B). Note the widened intestinal folds with increased cellularity in subepithelial tissues in fish fed the SBM diet as compared with fish fed the BPM-SBM diet.

### Conclusions

Morphological studies of the distal intestine show that the bacterial protein mean (BPM) prevents soybean meal induced enteritis.

### Example 5 - Feeding study

### Diets

Seven diets were formulated as described in Table 3; a fishmeal (FM) based control diet and six diets with inclusion of 200 g soybean meal (SBM) per kg feed and increasing amounts of bacterial protein meal (BPM).

The diets were produced using extrusion technology as previously described and were vacuum coated with fish oil. The resulting feed pellets were about 3.5 mm in diameter.

### Fish and rearing conditions

Atlantic salmon (*Salmo salar*) with an approximate 273g mean initial weight were randomly distributed into 14 tanks with 50 fish in each tank. The tanks were supplied with sea water, and the feeding experiment lasted for 6 weeks. All tanks were supplied with feed every 30 minutes, and excess feed was collected from the outlet water. The fish were fed about 20% in excess to ensure maximal feed intake. Excess feed, oxygen, temperature and unusual incidents were recorded daily according to standard routines. All fish were fed the FM based control diet for three weeks prior to the experiment to avoid possible interactions between the experimental diets and the commercial diets used at the research station.

### Weighing and sampling for analysis

The fish were weighed at the start and at termination of the feeding experiment. All fish were anaesthetised with MS222 and killed by a sharp blow to the head before the sampling at termination of the experiment.

The organs from six fish per tank were sampled for morphological and immunohistochemical studies. The gastrointestinal tract and liver of ten fish per tank were weighed, and the gastrointestinal tract was divided into the following sections: stomach, pyloric region, mid intestine and distal intestine. The faeces from 50 fish per tank was collected and pooled for determination of nutrient digestibility.

Morphological evaluation of the distal intestine was performed according to a method based on that described in Baeverfjord and Krogdahl (supra) including evaluation of the following four parameters:
1) Accumulation of leukocytes (lymphocytes, granulocytes and granular cells) in the lamina propria;
2) Changes in epithelium including a) Reduced vacuolization; b) Cytoplasmic basophilia (indicative of increased RNA); and c) Reduced cellular height
3) Atrophy, i.e. reduced height of the intestinal folds; and
4) Oedema, i.e. accumulation of protein-rich fluid in the lamina propria.

Individual histological sections were evaluated with a grading from 0 (no signs of intestinal changes) to 2 (marked signs of intestinal changes). Fish with a grading of 1 or less are generally considered to be within the normal variation expected in healthy fish.

### Results

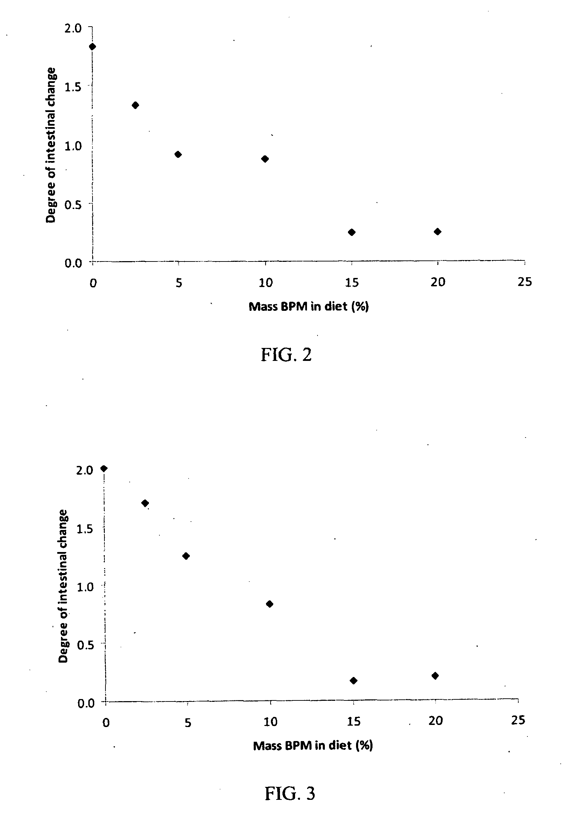

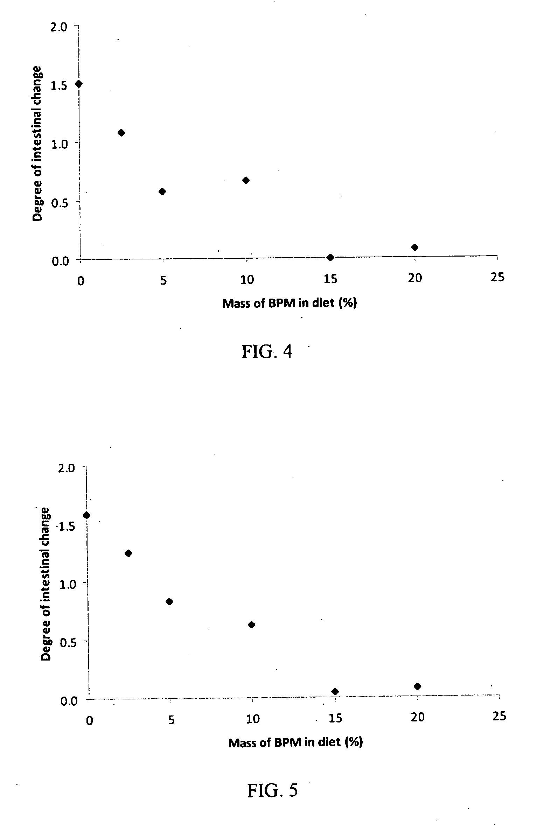

Results of the morphological and histological examination of distal intestines are shown in Figures 2 through 5. The data in these figures are mean average values from two independent fish tanks, i.e. duplicate tanks were used for each diet. Figure 2 shows the reduction in leukocytes accumulation in the lamina propria on feeding BPM. Figure 3 shows the protective effect of BPM on the epithelium. Figure 4 shows the changes in the intestinal folds (reduced atrophy) on increasing BPM in the diet. Figure 5 shows the reduction in oedema on increasing BPM levels in the diet.

Results also suggest no significant effects on growth, digestibility of lipids and starch or feed efficiency between the different diets.

Inclusion of BPM in the diets significantly increased (P<0.001) the weight of the distal intestine, based on the weight of distal intestine as a proportion of the total body weight, whereas the weight of the other sections of the gastrointestinal tract or liver was not significantly affected by diet.

### Conclusions

These data suggest that the protective effect of BPM is determined by the inclusion level in the feed. No morphological signs indicative of enteritis (classified as a degree of intestinal changes of greater than 1) were found in the distal intestines of Atlantic salmon fed 10% or more BPM in their diet (diets 3-7) and signs indicative of enteritis were only observed in the epithelia (Figure 3) of fish fed 5% BPM in the diet. The data suggest that inclusion of bacterial protein meal in the diet at a ratio of from 4:1 SBM:BPM is protective against soybean meal-induced enteritis in Atlantic salmon.

## Claims

1. A biomass derived from a culture of methanotrophic bacteria for use in the prevention or amelioration of plant-induced enteritis in fish.

2. A fish feed for use in the prevention or amelioration of plant-induced enteritis in fish, said fish feed comprising a biomass derived from a culture of methanotrophic bacteria.

3. The biomass or fish feed for use according to claim 1 or claim 2, wherein the plant-induced enteritis is soybean-induced enteritis.

4. The biomass or fish feed for use according to of any one of claims 1 to 3, wherein said biomass is derived from a microbial culture comprising a combination of methanotrophic and heterotrophic bacteria.

5. The biomass or fish feed for use according to claim 4, wherein the biomass is derived from a microbial culture comprising a combination of Methylococcus capsulatus (Bath) (strain NCIMB 41526) and the heterotrophic bacteria DB3 (strain NCIMB 41527) and DB5 (strain NCIMB 41525), optionally in combination with DB4 (strain NCIMB 41528).

6. The biomass or fish feed for use according to any one of claims 1 to 5, wherein the biomass is derived by one or more of the following processes: fermentation, centrifugation, filtration (e.g. ultrafiltration), homogenisation, hydrolysis and/or autolysis.

7. The biomass or fish feed for use according to any one of claims 1 to 6, wherein the biomass is a biomass concentrate.

8. The biomass or fish feed for use according to any one of claims 1 to 7, for feeding to salmonid fish, e.g. salmon or trout.

9. A feed component consisting essentially of a biomass derived from a culture of methanotrophic bacteria in combination with an enteritis-causing plant material.

10. The feed component of claim 9, wherein the enteritis-causing plant material is soybean meal.

11. The feed component of claim 9 or claim 10, wherein the ratio of said plant material to said biomass is less than 8:1, preferably between 4:1 and 1:1, especially about 2:1.

12. The feed component of any one of claims 9 to 11, wherein said biomass is as defined in any one of claims 4 to 7.

13. Use of a biomass as defined in any one of claims 1 to 12 in the manufacture of an agent for the prevention or amelioration of plant-induced enteritis in fish.

14. A process for preparing the feed component of any one of claims 9 to 12, which process comprises admixing a biomass derived from a culture of methanotrophic bacteria and an enteritis-causing plant material.

## Patentansprüche

1. Biomasse, die aus einer Kultur methanotropher Bakterien stammt, zur Verwendung für die Vorbeugung oder Verbesserung von pflanzeninduzierter Enteritis bei Fischen.

2. Fischfutter zur Verwendung für die Vorbeugung oder Verbesserung von pflanzeninduzierter Enteritis bei Fischen, wobei das Fischfutter eine Biomasse umfasst, die aus einer Kultur von methanotrophen Bakterien stammt.

3. Biomasse oder Fischfutter zur Verwendung nach Anspruch 1 oder Anspruch 2, wobei die pflanzeninduzierte Enteritis durch Sojabohnen induzierte Enteritis ist.

4. Biomasse oder Fischfutter zur Verwendung nach einem der Ansprüche 1 bis 3, wobei die Biomasse aus einer mikrobiologischen Kultur stammt, welche eine Kombination aus methanotrophen und heterotrophen Bakterien umfasst.

5. Biomasse oder Fischfutter zur Verwendung nach Anspruch 4, wobei die Biomasse aus einer mikrobiologischen Kultur stammt, die eine Kombination aus Methylococcus capsulatus (Bath) (Stamm NCIMB 41526) und den heterotrophen Bakterien DB3 (Stamm NCIMB 41527) und DB5 (Stamm NCIMB 41525), wahlweise in Kombination mit DB4 (Stamm NCIMB 41528), umfasst.

6. Biomasse oder Fischfutter zur Verwendung nach einem der Ansprüche 1 bis 5, wobei die Biomasse durch eines oder mehrere der folgenden Verfahren erhalten wird: Fermentation, Zentrifugation, Filtration (z. B. Ultrafiltration), Homogenisation, Hydrolyse und/oder Autolyse.

7. Biomasse oder Fischfutter zur Verwendung nach einem der Ansprüche 1 bis 6, wobei die Biomasse ein Biomassenkonzentrat ist.

8. Biomasse oder Fischfutter zur Verwendung nach einem der Ansprüche 1 bis 7 zum Füttern von Lachsfischen, z. B. Lachs oder Forelle.

9. Futterbestandteil, das im Wesentlichen aus einer Biomasse besteht, die aus einer Kultur von methanotrophen Bakterien in Kombination mit einem Enteritis verursachenden Pflanzenmaterial stammt.

10. Futterbestandteil nach Anspruch 9, wobei das Enteritis verursachende Pflanzenmaterial Sojamehl ist.

11. Futterbestandteil nach Anspruch 9 oder Anspruch 10, wobei das Verhältnis des Pflanzenmaterials zu der Biomasse weniger als 8:1, vorzugsweise zwischen 4:1 und 1:1, insbesondere etwa 2:1 beträgt.

12. Futterbestandteil nach einem der Ansprüche 9 bis 11, wobei die Biomasse wie in einem der Ansprüche 4 bis 7 definiert ist.

13. Verwenden einer Biomasse, wie in einem der Ansprüche 1 bis 12 definiert, bei der Herstellung eines Mittels für die Vorbeugung oder Verbesserung von pflanzeninduzierter Enteritis bei Fischen.

14. Verfahren zur Herstellung der Futterkomponente nach einem der Ansprüche 9 bis 12, wobei das Verfahren das Vermischen einer Biomasse, die aus einer Kultur methanotropher Bakterien stammt, mit einem Enteritis verursachenden Pflanzenmaterial umfasst.

## Revendications

1. Biomasse tirée d'une culture de bactéries méthanotrophes pour usage dans la prévention ou l'amélioration de l'entérite induite par des plantes chez le poisson.

2. Alimentation pour poissons pour usage dans la prévention ou l'amélioration de l'entérite induite par des plantes chez le poisson, ladite alimentation pour poissons comprenant une biomasse tirée d'une culture de bactéries méthanotrophes.

3. Biomasse ou alimentation pour poissons pour usage selon la revendication 1 ou la revendication 2, dans laquelle l'entérite induite par des plantes est l'entérite induite par le soja.

4. Biomasse ou alimentation pour poissons pour usage selon l'une quelconque des revendications 1 à 3, dans laquelle ladite biomasse est tirée d'une culture microbienne comprenant une combinaison de bactéries méthanotrophes et hétérotrophes.

5. Biomasse ou alimentation pour poissons pour usage selon la revendication 4, dans laquelle la biomasse est tirée d'une culture microbienne comprenant une combinaison de Methylococcus capsulatus (Bath) (souche NCIMB 41526) et des bactéries hétérotrophes DB3 (souche NCIMB 41527) et DB5 (souche NCIMB 41525), éventuellement en combinaison avec la DB4 (souche NCIMB 41528).

6. Biomasse ou alimentation pour poissons pour usage selon l'une quelconque des revendications 1 à 5, dans laquelle ladite biomasse est obtenue par un ou plusieurs des procédés suivants : fermentation, centrifugation, filtration (par exemple, ultrafiltration), homogénéisation, hydrolyse et/ou autolyse.

7. Biomasse ou alimentation pour poissons pour usage selon l'une quelconque des revendications 1 à 6, dans laquelle la biomasse est un concentré de biomasse.

8. Biomasse ou alimentation pour poissons pour usage selon l'une quelconque des revendications 1 à 7, pour l'alimentation de salmonidés, par exemple de saumons ou de truites.

9. Composant d'alimentation constitué essentiellement d'une biomasse tirée d'une culture de bactéries méthanotrophes en combinaison avec une matière végétale provoquant l'entérite.

10. Composant d'alimentation selon la revendication 9, dans lequel la matière végétale provoquant l'entérite est la farine de soya.

11. Composant d'alimentation selon la revendication 9 ou la revendication 10, dans lequel le rapport de ladite matière végétale à ladite biomasse est inférieur à 8:1, de préférence entre 4:1 et 1:1, en particulier d'environ 2:1.

12. Composant d'alimentation selon l'une quelconque des revendications 9 à 11, dans lequel ladite biomasse est telle que définie dans l'une quelconque des revendications 4 à 7.

13. Utilisation d'une biomasse selon l'une quelconque des revendications 1 à 12 dans la fabrication d'un agent pour la prévention ou l'amélioration de l'entérite induite par des plantes chez le poisson.

14. Procédé de préparation du composant d'alimentation selon l'une quelconque des revendications 9 à 12, lequel procédé comprend le mélange d'une biomasse tirée d'une culture de bactéries méthanotrophes et d'une matière végétale provoquant l'entérite.
